# EUROPEAN PATENT APPLICATION

(11) **EP 1 604 672 A1**
(43) Date of publication of application: **14.12.2005**
(21) Application number: 03707203.0
(22) Date of filing: 04.03.2003
(51) Int. Cl.: A61K 33/00, A61K 7/48, A61P 17/00

(54) **METHOD OF TRANSDERMAL AND TRANSMUCOSAL ABSORPTION OF CARBON DIOXIDE AND COSMETIC METHOD AND THERAPEUTIC METHOD**

(71) Applicant: Neochemir Inc., Kobe-shi, Hyogo 651-0087 (JP)
(72) Inventor: TANAKA, Masaya, Kobe-shi, Hyogo 654-0036 (JP)
(74) Representative: Sajda, Wolf E.
(86) International application number: PCT/JP2003/002523
(87) International publication number: WO 2004/078185

(57) **Abstract**

The present invention provides a method whereby cosmetic and therapeutic effects from carbon dioxide absorbed transdermally and transmucosally can be easily and strongly obtained. In this method, treatment to increase the amount of transdermal and transmucosal absorption of carbon dioxide is applied before or during carbon dioxide administration. Specifically, treatment is performed by applying a carbon dioxide absorption auxiliary containing a liquid for dissolving carbon dioxide which is at least one selected from the group consisting of water, lipids and organic solvents to the skin and mucous membranes. Alternatively, treatment is performed to lower the temperature of the skin and mucous membranes.

## Description

### TECHNICAL FIELD

The present invention relates to a transdermal and transmucosal absorption method of carbon dioxide, and to a cosmetic method and therapeutic method using same.

### BACKGROUND ART

Carbonated spring water has long been known to exhibit various cosmetic and therapeutic effects. Because these effects are achieved through transdermal and transmucosal absorption of carbon dioxide, artificial carbonated water producing bathing agent have been developed in an effort to achieve these effects easily, and have become readily available for household use. However, because of the low concentration of carbon dioxide gas among other reasons, (Hidenori Yorozu, "Studies of artificial carbonated water (3): Fundamental issues in preparing artificial carbonated water and the effects of dispersed carbon dioxide gas concentration," *Nichionkibutsu Ishi* 48, 79-85, 1985), satisfactory cosmetic and therapeutic effects have still to be achieved.

Moreover, a tabletop device for producing carbonated water is disclosed in Japanese Patent Application Laid-open No. H8-281087, in which it is disclosed that the carbonated water produced by this device has therapeutic effects in improving skin inflammations such as acne and the like. However, problems include the fact that carbonated water is laborious to produce, with large quantities of water and carbon dioxide gas being required to obtain adequate effects.

In addition, a composition for transdermal and transmucosal absorption of carbon dioxide is disclosed in Japanese Patent Application Laid-open No. 2000-319187. In this application it is disclosed that transdermal and transmucosal absorption of carbon dioxide is effective for itching in association with mucocutaneous diseases and mucocutaneous disorders such as athlete's foot, insect bites, atopic dermatitis, nummular eczema, xeroderma, seborrheic eczema, urticaria, prurigo, housewives' eczema, acne vulgaris, impetigo, folliculitis, carbuncles, furunculosis, phlegmon, pyoderma, psoriasis, ichthyosis, palmoplantar keratoderma, lichen, pityriasis, wounds, burns, rhagades, erosion, chilblain and the like; for mucocutaneous injuries such as decubitus ulcers, wounds, burns, angular stomatitis, stomatitis, skin ulcers, rhagades, erosion, chilblain and gangrene; for incomplete take of skin grafts, skin flaps and the like; for dental diseases such as gingivitis, alveolar pyorrhea, denture ulcers, nigricans gingiva and stomatitis; for skin ulcers, cryesthesia and numbness due to peripheral circulatory disorders such as thromboangiitis obliterans, arteriosclerosis obliterans, diabetic peripheral circulatory disorders, varicosis in lower extremities and the like; for musculoskeletal diseases such as chronic rheumatoid arthritis, cervico-omo-brachial syndrome, myalgia, arthralgia and lumbago; for nervous system diseases such as neuralgia, polyneuritis and subacute myelo-optic neuropathy; for keratoses including psoriasis, corns, callosities, ichthyosis, palmoplantar keratoderma, lichen and pityriasis; for suppurative dermopathies such as acne vulgaris, impetigo, folliculitis, carbuncles, furunculosis, phlegmon, pyoderma, suppurative eczema and the like; for suppressing hair regrowth after depilation (treatment of unwanted hair); for cosmetic problems of the skin, hair and the like such as freckles, chapped skin, dull complexion, loss of skin tautness or skin luster, loss of hair luster and the like; and for partial obesity. However, because even with this composition the amount of transdermal and transmucosal absorption of carbon dioxide is not very great, long-term continuous use is required to obtain satisfactory cosmetic and therapeutic effects.

In light of these issues, it is an object of the present invention to provide a transdermal and transmucosal absorption method of carbon dioxide capable of easily providing strong cosmetic and therapeutic effects from carbon dioxide absorbed transdermally and transmucosally, as well as a cosmetic method and a therapeutic method.

### DISCLOSURE OF THE INVENTION

The inventor in this case performed exhaustive research aimed at developing a technology capable of easily providing strong cosmetic and therapeutic effects from carbon dioxide absorbed transdermally and transmucosally. As a result, the inventor perfected the present invention upon discovering that cosmetic and therapeutic effects stronger than those obtained by administration of carbon dioxide using simply carbonated water or an external preparation containing carbon dioxide could be obtained if the amount of transdermal and transmucosal absorption of carbon dioxide was increased by applying, either before or during administration of carbon dioxide to the skin and mucous membranes, either a treatment to wet the skin and mucous membranes using a carbon dioxide absorption auxiliary containing at least a liquid for dissolving carbon dioxide selected from the group consisting of water, lipids and organic solvents, or a treatment to lower the temperature of the skin and mucous membranes.

That is, the transdermal and transmucosal absorption method of carbon dioxide of the present invention is a method of administering carbon dioxide to the skin and mucous membranes and causing the carbon dioxide to be absorbed transdermally and transmucosally, wherein a treatment is applied before or during the administration of carbon dioxide to increase the amount of transdermal and transmucosal absorption of carbon dioxide (Claim 1).

In this configuration, because treatment to increase the amount of transdermal and transmucosal absorption of carbon dioxide is applied to the skin and mucous membranes before or during administration of carbon dioxide, stronger cosmetic and therapeutic effects from carbon dioxide absorbed transdermally and transmucosally are obtained than with simple administration of carbonated water or an external preparation containing carbon dioxide.

"Carbon dioxide" here includes gaseous carbon dioxide as well as carbon dioxide dissolved in water or another solvent.

The aforementioned treatment is preferably a treatment to wet the skin and mucous membranes with a carbon dioxide absorption auxiliary having a liquid for dissolving carbon dioxide which is at least one selected from the group consisting of water, lipids and organic solvents as an essential component (Claim 2).

Carbon dioxide is known to be absorbed transdermally and transmucosally, but normally only a tiny amount is absorbed. When carbon dioxide is absorbed transdermally and transmucosally it must normally pass through the corneal layer first. Although the corneal layer, which is the outermost part of the skin and mucous membranes, already contains some water and lipids, the amount is less than in the cells below the corneal layer, and because corneal cells are not live cells they are deficient in active moisture and lipid retention functions. Because absorption of carbon dioxide into the corneal layer is the rate-limiting step for transdermal and transmucosal absorption of carbon dioxide, it is necessary to improve the ability of the corneal layer to absorb carbon dioxide in order to increase absorption of carbon dioxide and achieve therapeutic and cosmetic effects with carbon dioxide. This can be achieved by incorporating a liquid for dissolving carbon dioxide into the corneal layer. The liquid contains one or more selected from the group consisting of water, lipids and organic solvents in which carbon dioxide dissolves. Specifically, the skin and mucous membranes can be wetted, either before or during administration of carbon dioxide, with a liquid for dissolving carbon dioxide using a carbon dioxide absorption auxiliary (here and below, intended to include both carbon dioxide absorption auxiliary in liquid form and carbon dioxide absorption auxiliary formed in specific forms) having a liquid for dissolving carbon dioxide as an essential component.

There are no particular limits on the method of incorporating water into the corneal layer, but for example a method of immersing the part where the desired effects of carbon dioxide are to be obtained in water or a method of spraying it with water using an atomizer or the like can be used. A method of covering with a wet towel or wet tissue can also be used. This is desirable because the water does not drip and it is convenient to apply, the site is not particularly selected. It is also more desirable to prepare a carbon dioxide absorption auxiliary using appropriate raw materials which promote incorporation of water into the corneal layer, and apply it to the skin and mucous membranes. Although the degree to which water is incorporated into the skin and mucous membranes differs depending on the method of incorporation, the condition of the subject's skin and the like, more is better as long as there is no damage or the like to the skin and mucous membranes.

Methods which can be used to incorporate lipids into the corneal layer include, for example, a method of spreading lipids directly on the skin and mucous membranes, or a method of applying gauze or the like impregnated with lipids. Examples of lipids include fatty acids, neutral lipids having fatty acids bound with glycerol, and complex lipids such as phospholipids, cholesterol and the like, one or more of which can be used. When solid lipids are used they are dissolved in water or an organic solvent for purposes of use. A carbon dioxide absorption auxiliary can also be prepared using appropriate raw materials which promote incorporation of lipids into the corneal layer, and applied to the skin and mucous membranes. Although the degree to which lipids are incorporated into the skin and mucous membranes differs depending on the method of incorporation, the condition of the subject's skin and the like, more is better as long as there is no damage or the like to the skin and mucous membranes.

Methods which can be used to incorporate an organic solvent into the corneal layer include, for example, a method of directly spreading an organic solvent on the skin and mucous membranes, a method of spraying an organic solvent on the skin and mucous membranes and a method of applying gauze or the like impregnated with an organic solvent. Examples of organic solvents include ethanol, butanol and other alcohols and the like, one or more of which can be used. Appropriate raw materials can also be mixed with the organic solvent to promote incorporation into the corneal layer. Although the degree to which the organic solvent is incorporated into the skin and mucous membranes differs depending on the method of incorporation, the condition of the subject's skin and the like, more is better as long as there is no damage or the like to the skin and mucous membranes.

Of course, water, lipids and organic solvents can be used in combination, or can be used in combination with other raw materials as necessary.

When gaseous carbon dioxide or an external preparation containing carbon dioxide is used to administer carbon dioxide to skin or mucous membranes which have been wetted using a carbon dioxide absorption auxiliary having as an essential component a liquid for dissolving carbon dioxide which is at least one selected from the group consisting of water, lipids and organic solvents, carbon dioxide is easily absorbed as far as the subcutaneous tissue, and powerful therapeutic and cosmetic effects are easily obtained.

Treatment to lower the temperature of the skin and mucous membranes is desirable as the aforementioned treatment (Claim 3). Carbon dioxide is absorbed as it is dissolved in body fluid and the like of the skin and mucous membranes, and more carbon dioxide is dissolved the lower the temperature of the solvent. Consequently, transdermal and transmucosal absorption of carbon dioxide can be increased and the therapeutic and cosmetic effects of transdermal and transmucosal carbon dioxide absorption enhanced by lowering the temperature of the skin and mucous membranes.

There are no particular limits on the method of lowering the temperature of the skin and mucous membranes, which may be, for example, a method of pressing ice or a frozen cold insulator against the skin and mucous membranes, a method of applying cold air, or a method of administering a volatile substance such as an alcohol which takes vaporization heat from the skin and mucous membranes as it evaporates, lowering the temperature of the skin and mucous membranes. Although the degree to which the temperature of the skin and mucous membranes is lowered differs depending on the constitution of the subject, the temperature of the room and the like, normally the lower the temperature the better as long as there is no damage or the like to the skin and mucous membranes.

When gaseous carbon dioxide or an external preparation containing carbon dioxide is used to apply carbon dioxide to skin and mucous membranes the temperature of which has been lowered in this way, transdermal and transmucosal absorption of carbon dioxide is increased. As a result, greater cosmetic and therapeutic effects are obtained than when gaseous carbon dioxide or an external preparation containing carbon dioxide is used alone.

It is desirable to use a carbon dioxide absorption auxiliary which is set to a temperature lower than that of the skin and mucous membranes as the aforementioned carbon dioxide absorption auxiliary (Claim 4). In this case, the increase in carbon dioxide absorption due to the carbon dioxide absorption auxiliary and the increase in carbon dioxide absorption due to the lower temperature of the skin and mucous membranes work together to facilitate greater cosmetic and therapeutic effects.

It is desirable to use a liquid carbon dioxide absorption auxiliary containing the aforementioned liquid for dissolving carbon dioxide as the aforementioned carbon dioxide absorption auxiliary (Claim 5). This is cost-effective because the desired effects can be obtained with a small amount of liquid carbon dioxide absorption auxiliary. The liquid carbon dioxide absorption auxiliary may consist of the liquid for dissolving carbon dioxide alone or may be a mixed liquid to which other raw materials have been added.

It is desirable to use a carbon dioxide absorption auxiliary body containing the aforementioned liquid for dissolving carbon dioxide as the aforementioned carbon dioxide absorption auxiliary (Claim 6). This has the advantage that the liquid for dissolving carbon dioxide can be supplied continuously.

The carbon dioxide absorption auxiliary body here is one in which a molded body with a specific form capable of being impregnated or the like with the liquid for dissolving carbon dioxide is impregnated or the like with the liquid for dissolving carbon dioxide. An example is a wet polymer film. Wet polymer films are easy to handle and the water therein is unlikely to leak out, making them convenient for supplying moisture continuously to the corneal layer by simple application to the skin and mucous membranes. Moreover, because the wet polymer film itself functions as a carbon dioxide permeation membrane it helps compensate for the low carbon dioxide absorbability of the corneal layer. The greater the amount of water contained in the wet polymer membrane the better, with 15% by weight or more or preferably 30% by weight or more or more preferably 50% by weight or more of the total weight of the composition. The upper limit of water content differs depending on the polymer film used, but a higher content is better to the extent that the flexibility and other physical properties of the polymer film are not adversely affected. Moreover, a chilled wet polymer film provides stronger effects because it lowers the temperature while wetting the skin and mucous membranes.

There are no particular limitations on the wet polymer film used as long as it can form a film and contain water, and one or more selected from the group of natural polymers, semi-synthetic polymers and synthetic polymers can be used.

Examples of natural polymers include amylose, alginic acid, carageenan, karaya gum, chitin, xanthan gum, collagen, gelatin, dextran, starch, pullulan and the like, of which one or more can be used. Of these, carageenan and gelatin are desirable from the standpoint of feeling in use, affinity for skin and mucous membranes and the like.

Examples of semi-synthetic polymers include sodium alginate, propylene glycol alginate, carboxymethylcellulose, ethyl cellulose, starch-acrylic acid copolymer, hydroxypropyl cellulose, hydroxymethyl cellulose, methylcellulose and the like, of which one or more can be used. Of these, sodium alginate, propylene glycol alginate and starch-acrylic acid copolymer are preferred from the standpoint of feeling in use, affinity for skin and mucous membranes and the like.

Examples of synthetic polymers include acrylic acid-acrylamide copolymer, isobutylene-maleic anhydride copolymer, sulfonated polystyrene, polyacrylate/polysulfonate copolymer, polyvinylpyrrolidone, polyvinyl alcohol, polyacrylamide, polyacrylic acid, sodium polyacrylate, isopropyl acrylamide-butylmethacrylate copolymer, polyvinyl methyl ether, polyethylene glycol and the like, of which one or more can be used. Of these, polyvinylpyrrolidone, polyvinyl alcohol and polyacrylamide are preferred from the standpoint of feeling in use, affinity for skin and mucous membranes and the like.

The wet polymer membrane can include an aqueous solution or suspension of a thickener, and can also include raw materials commonly used in external preparations, such as perfumes, dyes, surfactants, oils, humectants, alcohols, preservatives, anti-oxidants, sequestering agents, anti-colorants, ultraviolet absorbers and scatterers, vitamins, amino acids, arbutin, kojic acid, nutrients, antiinflammatories, vasodilators, hormones, astringents, antihistamines, fungicides, corneum abraders and dissolvers, anti-seborrheics, sebum inhibitors, antipruritics and other chemicals.

It is desirable to use an absorption auxiliary further containing a thickener as the aforementioned carbon dioxide absorption auxiliary (Claim 7). The water or the like which is used as the liquid for dissolving carbon dioxide has high affinity for human tissue and is easy to handle, but by itself it is hard to stay on the skin and mucous membranes and hard to use as a carbon dioxide absorption auxiliary. By using a thickener to make the water viscous it is possible to ensure that it does not drip when applied to the skin and mucous membranes, resulting in more reliable carbon dioxide absorption through the skin and mucous membranes. An aqueous solution or suspension of a thickener may be applied as is to the skin and mucous membranes, or may be impregnated in woven or nonwoven cloth and used as a patch. An aqueous solution or suspension of the thickener forms a thin coat on the skin or mucous membrane, supplying moisture to the corneal layer and also functioning as a carbon dioxide permeation membrane so as to compensate for the low carbon dioxide absorbability of the corneal layer. Moreover, many thickeners such as hyaluronic acid and alginic acid also function as humectants, and when these are used it is possible to not only continuously wet the skin and mucous membranes but also to potentially achieve such effects as protection and humectation of the skin and mucous membranes. The quantity of a thickener in the carbon dioxide absorption auxiliary as a whole differs depending on the thickener, but can be a quantity sufficient to achieve viscosity so as to prevent dripping following application to the skin and mucous membranes.

At least one selected from the group consisting of natural polymers, semi-synthetic polymers, synthetic polymers and inorganic substances is preferably used as the aforementioned thickener (Claim 8).

Examples of natural polymers used as thickeners include plant polymers such as gum arabic, carageenan, galactan, agar, quince seed, guar gum, gum tragacanth, pectin, mannan, locust bean gum, wheat starch, rice starch, corn starch, potato starch and the like, microbial polymers such as curdlan, xanthan gum, succinoglucan, dextran, hyaluronic acid, pullulan and the like, protein polymers such as albumin, casein, collagen, gelatin, fibroin and the like, of which one or more can be used.

Examples of semi-synthetic polymers used as thickeners include cellulose type polymers such as ethyl cellulose, carboxymethyl cellulose and salts thereof, carboxymethylethyl cellulose and salts thereof, carboxymethyl starch and salts thereof, croscarmellose and salts thereof, crystalline cellulose, cellulose acetate, cellulose acetate phthalate, hydroxyethyl cellulose, hydroxypropyl cellulose, hydroxypropyl methylcellulose, hydroxypropyl methylcellulose phthalate, powdered cellulose, methyl cellulose, methyl hydroxypropyl cellulose and the like, starch type polymers such as pregelatinized starch, partly pregelatinized starch, carboxymethyl starch, dextrin, methylated starch and the like, alginic acid type polymers such as sodium alginate, propylene glycol alginate and the like, and other polysaccharide type polymers such as chondroitin sodium sulfate, sodium hyaluronate and the like, one or more of which can be used.

Examples of synthetic polymers used as thickeners include carboxyvinyl polymer, sodium polyacrylate, polyvinyl acetal diethylaminoacetate, polyvinyl alcohol, polyvinyl pyrrolidone, methacrylic acid-ethyl acrylate copolymer, methacrylic acid-ethyl methacrylate copolymer, ethyl methacrylate/trimethylammonium ethyl methacrylate chloride copolymer, dimethylaminoethyl methacrylate/methyl methacrylate copolymer and the like, one or more of which can be used.

Examples of inorganic substances used as thickeners include hydrated silicon dioxide, light anhydrous silicic acid, colloidal alumina, bentonite, laponite and the like, one or more of which can be used.

The aforementioned administration of carbon dioxide is preferably accomplished by administering gaseous carbon dioxide to the skin and mucous membranes (Claim 9). Gaseous carbon dioxide is a gas 5% or more of the total volume of which is carbon dioxide, and is easy to handle and relatively cheap. Gaseous carbon dioxide can be easily obtained of course in large-capacity cylinders, and also in small cylinders designed for aquatic plant culture or beer. It can also be produced from relatively cheap and readily available dry ice, or by reaction of carbonates and acids. Gaseous carbon dioxide can be blown as is on the skin and mucous membranes, but it is also possible to cover the skin and mucous membranes with a sealed container and fill that container with gaseous carbon dioxide, or to cover the skin and mucous membranes with a cover or the like and continuously pass gaseous carbon dioxide through that container. The administered amount of gaseous carbon dioxide differs depending on the concentration, the subject's skin condition and the like but is roughly proportional to administration time, and for example if the gaseous carbon dioxide consists of 100% carbon dioxide the administration time is 5 minutes to 6 hours or preferably 10 minutes to 3 hours or more preferably 15 minutes to 1 hour. If the administration time is less than 5 minutes adequate effects are not obtained, while if it exceeds 6 hours additional effects are not obtained.

The aforementioned gaseous carbon dioxide is preferably set to a temperature below that of the skin and mucous membranes (Claim 10). In this case, the amount of transdermal and transmucosal absorption of carbon dioxide is greater because the temperature of the skin and mucous membranes is lower during carbon dioxide administration. As a result, stronger desired effects from carbon dioxide can be easily obtained. The lower the temperature of the gaseous carbon dioxide the better as long as the skin and mucous membranes are not damaged.

The aforementioned administration of carbon dioxide is preferably accomplished by administering an external preparation containing carbon dioxide to the skin and mucous membranes (Claim 11).

The external preparation containing carbon dioxide is one which contains carbon dioxide whether as bubbles or dissolved in a solvent, and includes for example a composition containing gaseous carbon dioxide disclosed in Japanese Patent Application Laid-open No. 2000-319187 and other external compositions containing carbon dioxide, as well as carbon dioxide such as dry ice vapor which has been atomized by dissolution in water. Of these, an external composition containing carbon dioxide can administer carbon dioxide continuously and efficiently in small quantities because it retains relatively large quantities of carbon dioxide, and also offers the advantage of being convenient to transport. The administered amount of an external composition containing carbon dioxide differs depending on the carbon dioxide concentration, the subject's skin condition and the like but is roughly proportional to administration time, and the administration time is 5 minutes to 6 hours or preferably 10 minutes to 4 hours or more preferably 15 minutes to 3 hours. If the administration time is less than 5 minutes adequate effects are not obtained, while if it exceeds 6 hours additional effects are not obtained. Affixing a plastic film or other gas-impermeable film over an external composition containing carbon dioxide after application enhances the effects still more by preventing divergence of the carbon dioxide from the composition into the atmosphere. Strong cosmetic and therapeutic effects can also be easily obtained using dry ice vapor.

It is desirable to use an external preparation containing carbon dioxide which has been set to a temperature below that of the skin and mucous membranes as the aforementioned external preparation containing carbon dioxide (Claim 12). In this case, the amount of transdermal and transmucosal absorption of carbon dioxide is greater because the temperature of the skin and mucous membranes is lower during carbon dioxide administration. As a result, stronger desired effects from carbon dioxide can be easily obtained. Of course, the effects are further enhanced if the temperature of the skin and mucous membranes is lowered still further by laying a plastic film or other gas-impermeable film over the external preparation containing carbon dioxide and applying a cold insulator thereto. Specifically, for example if the external composition containing carbon dioxide has itself been cooled, transdermal and transmucosal absorption is increased at the site of application of the composition because the dermal and mucosal temperature of that site has been lowered. The cosmetic and therapeutic effects of the external composition containing carbon dioxide can thus be easily enhanced so that the desired effects are obtained in a short period of time. The temperature of the external composition containing carbon dioxide or the like should be as low as possible as long as it does not damage the skin or mucous membranes.

In the cosmetic method of the present invention, the aforementioned transdermal and transmucosal absorption method of carbon dioxide is applied (Claim 13). That is, in a cosmetic method of administering carbon dioxide to the skin and mucous membranes and causing it to be absorbed transdermally and transmucosally, a treatment is applied before or during the aforementioned carbon dioxide administration to increase the amount of transdermal and transmucosal absorption of carbon dioxide.

In this configuration, because treatment is applied to the skin and mucous membranes before or during carbon dioxide administration to increase the amount of transdermal and transmucosal absorption of carbon dioxide, the whitening and other cosmetic effects from carbon dioxide absorbed transdermally and transmucosally are greater than when carbonated water or an external preparation containing carbon dioxide is administered alone.

In the aforementioned cosmetic method, as described above, the aforementioned treatment is preferably treatment to wet the skin and mucous membranes with a liquid for dissolving carbon dioxide using a carbon dioxide absorption auxiliary having as an essential component a liquid for dissolving carbon dioxide which is at least one selected from the group of water, lipids and organic solvents. Moreover, the aforementioned treatment is preferably treatment to lower the temperature of the skin and mucous membranes. In addition, the aforementioned carbon dioxide absorption auxiliary is preferably one set to a temperature lower than that of the skin and mucous membranes. Moreover, it is desirable to use as the aforementioned carbon dioxide absorption auxiliary a carbon dioxide absorption auxiliary in liquid form comprising the aforementioned liquid for dissolving carbon dioxide. It is desirable to use as the aforementioned carbon dioxide absorption auxiliary a carbon dioxide absorption auxiliary body which contains the aforementioned liquid for dissolving carbon dioxide. It is desirable to use as the aforementioned carbon dioxide absorption auxiliary which further includes a thickener. At least one selected from the group of natural polymers, semi-synthetic polymers, synthetic polymers and inorganic substances is preferably used as the aforementioned thickener. The aforementioned administration of carbon dioxide is preferably accomplished by administering gaseous carbon dioxide to the skin and mucous membranes. The aforementioned administration of carbon dioxide is preferably accomplished by administering an external preparation containing carbon dioxide to the skin and mucous membranes. Gaseous carbon dioxide set to a temperature below that of the skin and mucous membranes is preferably used as the aforementioned gaseous carbon dioxide. An external preparation set to a temperature below that of the skin and mucous membranes is preferably used as the aforementioned external preparation containing carbon dioxide.

In the therapeutic method of the present invention, the aforementioned transdermal and transmucosal absorption method of carbon dioxide is applied (Claim 14). That is, in a therapeutic method of administering carbon dioxide to the skin and mucous membranes and causing it to be absorbed transdermally and transmucosally, a treatment is applied before or during the aforementioned carbon dioxide administration to increase the amount of transdermal and transmucosal absorption of carbon dioxide.

In this configuration, because treatment is applied to the skin and mucous membranes before or during carbon dioxide administration to increase the amount of transdermal and transmucosal absorption of carbon dioxide, the therapeutic effects for atopic dermatitis from carbon dioxide absorbed transdermally and transmucosally are greater than when carbonated water or an external preparation containing carbon dioxide is administered alone.

In the aforementioned cosmetic method, as described above, the aforementioned treatment is preferably treatment to wet the skin and mucous membranes with a liquid for dissolving carbon dioxide using a carbon dioxide absorption auxiliary having as an essential component a liquid for dissolving carbon dioxide which is at least one selected from the group of water, lipids and organic solvents. Moreover, the aforementioned treatment is preferably treatment to lower the temperature of the skin and mucous membranes. Moreover, the aforementioned carbon dioxide absorption auxiliary is preferably one set to a temperature lower than that of the skin and mucous membranes. Moreover, it is desirable to use as the aforementioned carbon dioxide absorption auxiliary a carbon dioxide absorption auxiliary in liquid form comprising the aforementioned liquid for dissolving carbon dioxide. It is desirable to use as the aforementioned carbon dioxide absorption auxiliary a carbon dioxide absorption auxiliary body which contains the aforementioned liquid for dissolving carbon dioxide. It is desirable to use as the aforementioned carbon dioxide absorption auxiliary a carbon dioxide absorption auxiliary which further includes a thickener. At least one selected from the group of natural polymers, semi-synthetic polymers, synthetic polymers and inorganic substances is preferably used as the aforementioned thickener. The aforementioned administration of carbon dioxide is preferably accomplished by administering gaseous carbon dioxide to the skin and mucous membranes. The aforementioned administration of carbon dioxide is preferably accomplished by administering an external preparation containing carbon dioxide to the skin and mucous membranes. Gaseous carbon dioxide set to a temperature below that of the skin and mucous membranes is preferably used as the aforementioned gaseous carbon dioxide. An external preparation set to a temperature below that of the skin and mucous membranes is preferably used as the aforementioned external preparation containing carbon dioxide.

### BEST MODE FOR CARRYING OUT THE INVENTION

The present invention is explained in more detail below using examples, but the present invention is not limited to these examples. In the following examples "carbon dioxide absorption auxiliary liquid" is abbreviated as "absorption auxiliary liquid" and "carbon dioxide absorption auxiliary body" as "absorption auxiliary body".

### Example 1

### (Test of whitening by gaseous carbon dioxide of the back of a hand treated with absorption auxiliary liquid)

Using water as the absorption auxiliary liquid, the right hand of a 22-year-old woman was soaked in 36°C water for 5 minutes to wet the hand. Leaving the left hand as is, each hand was encased in a vinyl bag 25 cm long by 20 cm wide, each wrist was tied with an elastic cord to seal it, and the air in the bags was removed. When a vinyl tube was inserted through the elastic cord enclosure of each bag and gaseous carbon dioxide from a carbon dioxide cylinder was blown in to inflate each vinyl bag as much as possible with gaseous carbon dioxide, augmented blood flow was observed in the back of the right hand, which immediately turned red, but no change was observed in the back of the left hand. 20 minutes later when the vinyl bags were removed, the back of the right hand lost its redness within about 2 minutes, at which point the skin appeared whiter than that of the back of the left hand, with more luster and a slight raising of the blood vessels, while almost no change in the back of the left hand was observed in comparison with its condition before the test.

### Example 2

### (Test of whitening by an external preparation containing carbon dioxide of the back of a hand treated with an absorption auxiliary liquid)

### [Preparation of carbon dioxide-containing external composition A]

Carbon dioxide-containing external composition A was prepared using 2.4 parts by weight of sodium bicarbonate, 2.0 parts by weight of sodium alginate, 2.0 parts by weight of carboxymethyl starch sodium, 91.6 parts by weight of purified water and 2.0 parts by weight of citric acid, following example 18 of Japanese Patent Application Laid-open No. 2000-319187.

### [Whitening test of back of hand]

Using water as the absorption auxiliary liquid, the right hand of a 22-year-old woman was soaked in 36°C water for 5 minutes to wet the hand. Leaving the left hand as is, 15 g of carbon dioxide containing external composition A was applied to the backs of both hands, and left for 15 minutes. 15 minutes later when the composition was removed augmented blood flow was observed, with the backs of both hands appearing red overall, but the redness had disappeared entirely after about 2 minutes. After the redness had completely disappeared the skin of the back of the right hand appeared whiter than that of the left hand, with more luster and a slight raising of the blood vessels, but the blood vessels of the back of the left hand did not appear raised.

### Example 3

### (Test of whitening by gaseous carbon dioxide of the back of a hand with lowered skin temperature)

The back of the right hand of a 22-year-old woman was cooled overall by application of a frozen cold insulator wrapped in a towel, lowering the skin temperature to 20°C. Leaving the left hand as is, each hand was encased in a vinyl bag 25 cm long by 20 cm wide, each wrist was tied with a elastic cord to seal it, and the air in the bags was removed. When a vinyl tube was inserted through the elastic cord enclosure of each bag and gaseous carbon dioxide from a carbon dioxide cylinder was blown in to inflate each vinyl bag as much as possible with gaseous carbon dioxide, augmented blood flow was observed in the back of the right hand, which immediately turned red, but no change was observed in the back of the left hand. 20 minutes later when the vinyl bags were removed, the back of the right hand lost its redness after about 2 minutes and the skin appeared whiter than that of the back of the left hand, with more luster and a slight raising of the blood vessels, while almost no change in the back of the left hand was observed in comparison with its condition before the test.

### Example 4

### (Test of whitening by a carbon dioxide-containing external preparation of the back of a hand with lowered skin temperature)

The back of the right hand of a 22-year-old woman was cooled overall by application of a frozen cold insulator wrapped in a towel, lowering the skin temperature to 20°C. Leaving the left hand as is, 15 g of carbon dioxide-containing external composition A was applied to the backs of both hands, and left for 15 minutes. 15 minutes later when the composition was removed augmented blood flow was observed, with the backs of both hands appearing red overall, but the redness had disappeared entirely after about 2 minutes. After the redness had completely disappeared the skin of the back of the right hand appeared whiter than that of the left hand, with more luster and a slight raising of the blood vessels, but the blood vessels of the back of the left hand did not appear raised.

### Example 5

### (Test of improvement in partial obesity of an upper arm using a cool carbon dioxide-containing external preparation)

100 g of carbon dioxide-containing external composition A cooled to 4°C was applied to the entire upper right arm and 100 g of carbon dioxide-containing external composition A at 36° C to the entire upper left arm of a 21-year-old woman who was concerned about the thickness of her upper arms, and food packaging film was wrapped around both arms and left for 30 minutes. After removal of the composition, the circumference of the middle of the upper right arm had shrunk from 28.5 cm before administration of carbon dioxide-containing external composition A to 27.0 cm, but there was no change in the circumference of the upper left arm. Adding to that, the skin of the upper right arm was whiter and more lustrous than the surrounding skin and the skin of the upper left arm.

### Example 6

### (Test of whitening by gaseous carbon dioxide of the back of a hand treated with a cool absorption auxiliary liquid)

Using water as the absorption auxiliary liquid, the right hand of a 22-year-old woman was simultaneously wetted overall and chilled by soaking for 3 minutes in water at 4°C, lowering the skin temperature to 20°C. Leaving the left hand as is, each hand was encased in a transparent vinyl bag 25 cm long by 20 cm wide, each wrist was tied with a elastic cord to seal it, and the air in the bags was removed. When a vinyl tube was inserted through the elastic cord enclosure of each bag and gaseous carbon dioxide from a carbon dioxide cylinder was blown in to inflate each vinyl bag as much as possible with gaseous carbon dioxide, augmented blood flow was observed in the back of the right hand, which immediately turned red, but no change was observed in the back of the left hand. 20 minutes later when the vinyl bags were removed, the back of the right hand lost its redness after about 2 minutes and the skin appeared whiter than that of the back of the left hand, with more luster and a slight raising of the blood vessels, while almost no change in the back of the left hand was observed in comparison with its condition before the test.

### Example 7

### (Test of partial slimming using a carbon dioxide-containing external preparation of a face treated with a cool absorption auxiliary body)

### [Preparation of carbon dioxide-containing external composition B]

218 g of basic composition were obtained using 200 ml of water, 2 g of sodium bicarbonate, 6 g of sodium alginate, 8 g of sodium carboxymethylcellulose, 2 g of 1,3-butylene glycol and a trace amount of sodium copper chlorophylline according to example 309 of Japanese Patent Application No. 2000-319187. Also according to example 309 of the same application, 80 kg of granules were obtained using 13.5 kg of citric acid, 5.4 kg of 7% HPC-L ethanol solution and 67 kg of purified sucrose granules. 25 g of the basic composition was mixed and shaken with 1.2 g of the granules until the granules were completely dissolved to obtain carbon dioxide-containing external composition B.

### [Partial facial slimming test]

A hand towel (impregnated with water, absorption auxiliary body) cooled to 4°C in the refrigerator was applied for 3 minutes to the right cheek of a 30-year-old woman who was concerned about her plump cheeks to wet the skin and lower the skin temperature to 20°C, and when carbon dioxide-containing external composition B was then applied to the entire face the right angle of the mouth began to rise 4 minutes after application and the right cheek began to be smaller than the left cheek. When carbon dioxide-containing external composition B was completely removed 20 minutes after application, the right angle of the mouth was clearly higher and the right cheek was smaller than the left cheek. The woman herself also experienced partial slimming as if her right cheek was being pulled and a skin tightening effect. Adding to that the right cheek was whiter and more lustrous than the rest of the face. Comparing photographs of the skin of the cheek taken at 50X magnification before and after application of carbon dioxide-containing external composition B, the mounds and grooves on the skin of the right cheek were much clearer after application than before, indicating an obvious rejuvenation pattern. Meanwhile, the mounds and grooves on the skin of the left cheek were also clearer than before application of the external preparation, but not to the same degree as on the right cheek.

### Example 8

### (Cosmetic test by application of a carbon dioxide-containing external preparation to a face treated with a cool absorption auxiliary body, followed by further lowering of the facial skin temperature)

A hand towel (impregnated with water, absorption auxiliary body) cooled to 4°C in the refrigerator was applied for 3 minutes to the right half of the face of a 42-year-old woman who was concerned about her plump cheeks, wetting the skin and lowering the skin temperature to 20°C, and 30 g of carbon dioxide-containing external composition A was then spread on the entire face and covered with food packaging film. A hand towel cooled to 4°C was then applied for 15 minutes over the film to the right half of the face to cool it still further, and when the aforementioned composition A was removed the right cheek was smaller than the left cheek and the position of the right eye was about 5 mm higher than the position of the left eye, indicating an obvious shrinking and lifting effect on the face. Moreover, the skin of the right half was whiter and finer than the skin of the left half.

### Example 9

### (Test of wound treatment using a cool carbon dioxide-containing agent)

When 2 g of carbon dioxide-containing external composition A cooled to 4°C was applied to a 1 cm long cut on the left shin of a 47-year-old man and left for 15 minutes, the wound closed completely. However, when 1 g of carbon dioxide-containing external composition A at 36°C was applied to a 5 mm long cut on the left heel of the same man and left for 15 minutes, the wound did not close.

### Example 10

### (Test of partial slimming by a cool carbon dioxide-containing agent of a face treated with a cool absorption auxiliary body)

A hand towel cooled to 4°C (impregnated with water, absorption auxiliary body) was applied for 3 minutes to the entire face of a 43-year-old woman to wet the skin and lower the skin temperature to 21°C, after which 15 g of carbon dioxide-containing external composition A cooled to 4°C was applied to the right half of the face and 15 g of carbon dioxide-containing external composition A at 36°C to the left half of the face, left for 10 minutes and completely removed. As a result, the right angle of the mouth rose, the right cheek was smaller than the left cheek, and the position of the right eye was about 4 mm higher than the position of the left eye. The woman herself felt that both cheeks were pulled and recognized a skin-tightening effect, with the pulling sensation being greater in the right cheek. The face was whiter and more lustrous overall, but the right side in particular appeared whiter and more lustrous than the left side.

### Example 11

### (Test of improvement by gaseous carbon dioxide in partial obesity of an upper arm treated with an absorption auxiliary liquid)

### [Preparation of absorption auxiliary liquid A]

1 part by weight of sodium alginate and 1 part by weight of sodium carboxymethylcellulose were added and dissolved in 98 parts by weight of purified water to obtain absorption auxiliary liquid A comprising a semi-synthetic polymer of pH 7.3.

### [Test of improvement in partial obesity of an upper arm]

0.5 g of absorption auxiliary liquid A was applied to the entire upper left arm of a 40-year-old woman who was concerned about the thickness of her upper arms, and left for 10 minutes to wet the arm. With the right upper arm untreated, vinyl sleeves 15 cm long and 38 cm in circumference were used to cover the entire upper part of both left and right arms and sealed at both ends with elastic cord, and the air was removed. When a vinyl tube was inserted through the elastic cord enclosure of each sleeve and gaseous carbon dioxide from a carbon dioxide cylinder was blown in to inflate each vinyl sleeve as much as possible with gaseous carbon dioxide, augmented blood flow was observed in the upper left arm, which immediately turned red, but no change was observed in the upper right arm. When the sleeves were removed after 30 minutes and the circumference of both upper arms measured at the center part, the upper left arm had shrunk from 33 cm before gaseous carbon dioxide administration to 32 cm, but no change was observed in the circumference of the upper right arm. The skin of the upper left arm had lost its redness by about 2 minutes after removal of the vinyl sleeve, no side-effects were observed, and the surrounding skin was whiter and more lustrous than that of the upper right arm.

### Example 12

### (Test of improvement by a cool carbon-dioxide containing external preparation in partial obesity of an abdomen applied an absorption auxiliary)

### [Preparation of wet polymer film A]

A 20 cm x 40 cm cellulose film was thoroughly impregnated with distilled water to prepare wet polymer film A as an absorption auxiliary body.

### [Preparation of dry ice vapor]

200 g of dry ice was placed in a closed container connected to a vinyl tube with an inner diameter of 1 cm, and water was applied as appropriate to produce dry ice vapor (cool carbon-dioxide containing external preparation).

### [Test of improvement in partial obesity of an abdomen]

When wet polymer film A was laid on the abdomen of a 37-year-old man who was concerned about abdominal obesity and wet polymer film A was showered for 20 minutes continuously with dry ice vapor through the vinyl tube the waist size shrank from 92 to 91 cm. Moreover, the skin covered by wet polymer film A was whiter and more lustrous than the other abdominal skin. The abdomen of a 34-year-old man who was also concerned about abdominal obesity was also showered for 40 minutes continuously with dry ice vapor from the vinyl tube, but no waist shrinkage or skin whitening effect was observed.

### Example 13

### (Test of improvement by gaseous carbon dioxide in spots of the back of a hand treated with a cool absorption auxiliary body)

### [Preparation of wet polymer film B]

A 10 cm x 20 cm cellulose film was thoroughly impregnated with distilled water to prepare wet polymer film B as the absorption auxiliary body.

### [Spot improvement test]

Wet polymer film B cooled to 4°C was laid on the back of the right hand of a 69-year-old woman who complained of conspicuous spots on the backs of her hands. Leaving the left hand as is, each hand was encased in a transparent vinyl bag 25 cm long by 20 cm wide, each wrist was tied with an elastic cord to seal it, and the air in the bags was removed. A vinyl tube was inserted through the elastic cord enclosure of each bag and gaseous carbon dioxide from a carbon dioxide cylinder was blown in to inflate each vinyl bag as much as possible with gaseous carbon dioxide, and left for 15 minutes. This treatment was repeated for a week, and the back of the right hand was whiter overall than the back of the left hand, and the spots were no longer conspicuous. The blood vessels were also slightly raised on the back of the right hand, but the back of the left hand was almost unchanged in comparison with its condition before the test.

### Example 14

### (Test of improvement by gaseous carbon dioxide in partial obesity of a thigh treated with a cool absorption auxiliary liquid)

### [Preparation of absorption auxiliary liquid B]

1 part by weight of carageenan was added and dissolved in 99 parts by weight of purified water to obtain absorption auxiliary liquid B comprising a natural polymer at pH 7.2.

### [Test of improvement in partial obesity of a thigh]

3 g of absorption auxiliary liquid B cooled to 4°C was applied to the entire right thigh of a 40-year-old woman who was concerned about the thickness of her thighs, and left for 3 minutes to wet the skin and lower its temperature to 21.5°C. With nothing applied to the left thigh, vinyl sleeves 20 cm long and 45 cm in circumference were used to entirely cover the left and right thighs and sealed at both ends with elastic cord, and the air was removed. When a vinyl tube was inserted through the elastic cord enclosure of each sleeve and gaseous carbon dioxide from a carbon dioxide cylinder was blown in to inflate each vinyl sleeve as much as possible with gaseous carbon dioxide, augmented blood flow was observed in the right thigh, which immediately turned red, but no change was observed in left thigh. When the sleeves were removed from both thighs after 20 minutes and the circumference of both thighs measured at the center part, the right thigh had shrunk from 41 cm before gaseous carbon dioxide administration to 40 cm, but no change was observed in the circumference of the left thigh. The skin of the right thigh lost its redness within about 2 minutes after removal of the vinyl sleeve, no side-effects were observed, and the surrounding skin was whiter and more lustrous than that of the left thigh.

### Example 15

### (Test of improvement by a carbon dioxide-containing external composition in spots and freckles of a face treated with a cool absorption auxiliary body)

Cut cotton (absorption auxiliary body) soaked in absorption auxiliary liquid A cooled to 4°C was applied for 5 minutes so as to cover the right half of the face of a 41-year-old woman concerned about conspicuous facial spots and freckles, while cut cotton (absorption auxiliary body) soaked in ordinary purified water cooled to 4°C was applied for 5 minutes so as to cover the left half of the face, wetting the skin and cooling it to 21°C. After the cotton was removed, 30 g of carbon dioxide-containing external composition A was spread on the entire face and left for 10 minutes as a pack. After this pack had been continued every day for 12 days, the right half of the face was whiter than the left half, and the spots and freckles were no longer conspicuous. Although the left half of the face was whiter than before the test, there was no improvement in spots or freckles, which were actually more conspicuous due to whitening of the skin.

### Example 16

### (Whitening test using gaseous carbon dioxide on the back of a hand treated with an absorption auxiliary liquid)

0.5 g of γ-linolenic acid (lipid) as the absorption auxiliary liquid was applied to the back of the right hand of a 27-year-old woman, and leaving the left hand as is, each hand was encased in a transparent vinyl bag 25 cm long by 20 cm wide, each wrist was tied with a elastic cord to seal it, and the air in the bags was removed. When a vinyl tube was inserted through the elastic cord enclosure of each bag and gaseous carbon dioxide from a carbon dioxide cylinder was blown in to inflate each vinyl bag as much as possible with gaseous carbon dioxide, augmented blood flow was observed in the back of the right hand, which immediately turned red, but no change was observed in the back of the left hand. 20 minutes later when the vinyl bags were removed, the back of the right hand lost its redness after about 2 minutes and the skin appeared whiter than that of the back of the left hand, with more luster and a slight raising of the blood vessels, while almost no change in the back of the left hand was observed in comparison with its condition before the test.

### Example 17

### (Whitening test using gaseous carbon dioxide on the back of a hand treated with an absorption auxiliary liquid)

0.5 g of n-butanol (organic solvent) as the absorption auxiliary liquid was applied to the back of the right hand of a 27-year-old woman, and leaving the left hand as is, each hand was encased in a transparent vinyl bag 25 cm long by 20 cm wide, each wrist was tied with a elastic cord to seal it, and the air in the bags was removed. When a vinyl tube was inserted through the elastic cord enclosure of each bag and gaseous carbon dioxide from a carbon dioxide cylinder was blown in to inflate each vinyl bag as much as possible with gaseous carbon dioxide, augmented blood flow was observed in the back of the right hand, which immediately turned red, but no change was observed in the back of the left hand. 10 minutes later when the vinyl bags were removed, the back of the right hand lost its redness after about 2 minutes and the skin appeared whiter than that of the back of the left hand, with more luster and a slight raising of the blood vessels, while almost no change in the back of the left hand was observed in comparison with its condition before the test.

### Example 18

### (Therapeutic test using gaseous carbon dioxide for atopic dermatitis of an arm treated with an absorption auxiliary liquid)

1 g of absorption auxiliary liquid A was applied to the atopic dermatitis of the right arm of a 9-year-old girl with atopic dermatitis. The atopic dermatitis of the left arm was not treated. A vinyl sleeve 15 cm long and 10 cm in diameter was used to cover each area of atopic dermatitis and tied at both ends with elastic cord, and the air was removed. Tubes were inserted in the aforementioned vinyl sleeves and 100% carbon dioxide gas was blown in from a carbon dioxide cylinder to inflate the sleeves. The skin of the right arm treated with absorption auxiliary liquid A immediately turned red and a vasodilation effect was confirmed, but there was no change to the skin of the left arm. When the vinyl sleeves were removed after 10 minutes, the skin with atopic dermatitis of the right arm was smoother and no longer itchy. There was no particular change to the skin with atopic dermatitis of the left arm, which was somewhat itchier than before.

### INDUSTRIAL APPLICABILITY

With the transdermal and transmucosal absorption method of carbon dioxide of the present invention, because more carbon dioxide is absorbed, the cosmetic and therapeutic effects described above from transdermal and transmucosal absorption of carbon dioxide can be obtained easily and strongly. Specifically, the cosmetic and therapeutic effects are stronger and more easily obtained than with carbonated spring water, the cosmetic and therapeutic effects are greater than with simple application of a carbon dioxide-containing external composition, and the desired effects are obtained in a short period of time. Consequently, the transdermal and transmucosal absorption method of carbon dioxide of the present invention can be favorably applied to cosmetic methods, therapeutic methods and the like.

## Claims

1. A transdermal and transmucosal absorption method of administering carbon dioxide to the skin and mucous membranes and causing the carbon dioxide to be absorbed transdermally and transmucosally, wherein a treatment is applied before or during the administration of carbon dioxide to increase the amount of transdermal and transmucosal absorption of carbon dioxide.

2. The transdermal and transmucosal absorption method of carbon dioxide according to Claim 1, wherein said treatment is a treatment to wet the skin and mucous membranes with a carbon dioxide absorption auxiliary having a liquid for dissolving carbon dioxide which is at least one selected from the group consisting of water, lipids and organic solvents as an essential component.

3. The transdermal and transmucosal absorption method of carbon dioxide according to Claim 1, wherein said treatment is a treatment to lower the temperature of the skin and mucous membranes.

4. The transdermal and transmucosal absorption method of carbon dioxide according to Claim 2, wherein a carbon dioxide absorption auxiliary set to a temperature lower than that of the skin and mucous membranes is used as said carbon dioxide absorption auxiliary.

5. The transdermal and transmucosal absorption method of carbon dioxide according to Claim 2, wherein a liquid carbon dioxide absorption auxiliary containing said liquid for dissolving carbon dioxide is used as the carbon dioxide absorption auxiliary.

6. The transdermal and transmucosal absorption method of carbon dioxide according to Claim 2, wherein a carbon dioxide absorption auxiliary body containing said liquid for dissolving carbon dioxide is used as said carbon dioxide absorption auxiliary.

7. The transdermal and transmucosal absorption method of carbon dioxide according to Claim 2, wherein a carbon dioxide absorption auxiliary further containing a thickener is used as said carbon dioxide absorption auxiliary.

8. The transdermal and transmucosal absorption method of carbon dioxide according to Claim 7, wherein at least one selected from the group consisting of natural polymers, semi-synthetic polymers, synthetic polymers and inorganic substances is used as said thickener.

9. The transdermal and transmucosal absorption method of carbon dioxide according to Claim 1, wherein said administration of carbon dioxide is accomplished by administering gaseous carbon dioxide to the skin and mucous membranes.

10. The transdermal and transmucosal absorption method of carbon dioxide according to Claim 9, wherein gaseous carbon dioxide set to a temperature below that of the skin and mucous membranes is used as said gaseous carbon dioxide.

11. The transdermal and transmucosal absorption method of carbon dioxide according to Claim 1, wherein said administration of carbon dioxide is accomplished by administering an external preparation containing carbon dioxide to the skin and mucous membranes.

12. The transdermal and transmucosal absorption method of carbon dioxide according to Claim 11, wherein an external preparation containing carbon dioxide set to a temperature below that of the skin and mucous membranes is used as said external preparation containing carbon dioxide.

13. A cosmetic method, wherein the transdermal and transmucosal absorption method of carbon dioxide according to any of Claims 1 through 12 is performed.

14. A therapeutic method, wherein the transdermal and transmucosal absorption method of carbon dioxide according to any of Claims 1 through 12 is performed.
